# EUROPÄISCHE PATENTSCHRIFT

(11) **EP 3 965 706 B1**
(45) Veröffentlichungstag und Bekanntmachung des Hinweises auf die Patenterteilung: **01.01.2025**
(21) Anmeldenummer: 20713214.3
(22) Anmeldetag: 13.03.2020
(51) Int. Cl.: A61F 5/02, A61H 1/02

(54) **VORRICHTUNG ZUR UNTERSTÜTZUNG VON BEWEGUNGEN UND HEBEBEWEGUNGEN DES MENSCHLICHEN KÖRPERS**
DEVICE FOR SUPPORTING MOVEMENTS AND LIFTING MOVEMENTS OF THE HUMAN BODY
DISPOSITIF POUR ACCOMPAGNER DES MOUVEMENTS ET DES MOUVEMENTS DE LEVAGE DU CORPS HUMAIN

(30) Priorität: 06.05.2019 DE 102019111718
(43) Veröffentlichungstag der Anmeldung: 16.03.2022
(73) Patentinhaber: Mast, Jonas, 72270 Baiersbronn (DE)
(72) Erfinder: MAST, Jonas, 72270 Baiersbronn (DE); BURGHARDT, Joshua, 70193 Stuttgart (DE); PONZONI, Eduardo Dognini, 70193 Stuttgart (DE)
(74) Vertreter: Westphal, Mussgnug & Partner Patentanwälte mbB
(86) Internationale Anmeldenummer: PCT/EP2020/056928
(87) Internationale Veröffentlichungsnummer: WO 2020/224836

(56) Entgegenhaltungen:
- US-A1- 2015 366 694
- US-A1- 2018 071 129

## Beschreibung

Die Erfindung betrifft eine Vorrichtung zur Unterstützung von Bewegungen und Hebebewegungen des menschlichen Körpers, welche am Körper tragbar ist.

Viele Personen, insbesondere gewerbliche Arbeitnehmer, sind bei unterschiedlichen Tätigkeiten hohen physischen Belastungen ausgesetzt. Hierbei wird insbesondere der Rücken beim Heben und Tragen von Lasten, durch ungünstige Haltungen oder sich ständig wiederholende manuelle Tätigkeiten stark belastet. Um hier eine Unterstützung zu ermöglichen, sind Vorrichtungen zur Unterstützung von Bewegungen und Hebebewegungen des menschlichen Körpers, welche am Körper tragbar sind, bekannt. Derartige Vorrichtungen werden auch als Exoskelette bezeichnet.

Dabei sind aktive und passive Exoskelette bekannt. Aktive Exoskelette zeichnen sich dadurch aus, dass sie elektrische Motoren, hydraulische Komponenten oder ähnliche Systeme aufweisen, die Bewegungen der Gelenke des menschlichen Körpers durch ihren Antrieb aktiv unterstützen können. Die Person erfährt somit eine direkte Unterstützung bei ihren Bewegungen und Tätigkeiten. Aktive Exoskelette weisen aufgrund der Maschinenelemente jedoch ein hohes Eigengewicht auf. Zudem ist die Abstimmung des Antriebs der Maschinenelemente auf den Bewegungsvorgang nicht einfach, da die Maschinenelemente erkennen müssen, wann welcher Bewegungsvorgang durchgeführt wird. Schließlich tragen derartige aktive Exoskelette vom Bauraum her auf und sind unhandlich zu handhaben.

Passive Exoskelette weisen keinen Antrieb auf, sondern speichern potentielle Energie bei einer Flexion des Körpers oder des entsprechenden Gelenks. Bei einer Reflexion wird diese Energie wieder freigegeben und unterstützt die entsprechende Bewegung. Das Eigengewicht von passiven Exoskeletten ist in der Regel geringer als das von aktiven Exoskeletten, und üblicherweise trägt das passive Exoskelett weniger auf als das aktive Exoskelett.

Aus der DE 10 2017 123 518 A1 ist beispielsweise ein passives Exoskelett mit einem Hüftelement, mit zwei Schulterbändern, mit einem Rückenelement, welches an dem Hüftelement angeordnet ist, und mit zwei Beinbändern, welche derart angeordnet sind, dass sie im an den Körper angelegten Zustand der Vorrichtung am Gesäß des Körpers entlang verlaufen, bekannt.

Diese Vorrichtung weist den Nachteil auf, dass durch den Zug der Beinbänder an der Hüfte das Becken der die Vorrichtung tragenden Person nach hinten gekippt wird, was die Gefahr einer Neigung zu einer Hyperkyphose oder anders ausgedrückt einem Rundrücken birgt.

Die Aufgabe der Erfindung besteht daher darin, eine Vorrichtung zur Unterstützung von Bewegungen und Hebebewegungen des menschlichen Körpers, welche am Körper tragbar ist, insbesondere ein passives Exoskelett, derart weiterzubilden, dass die Stabilisierung der Wirbelsäule verbessert wird.

Die Aufgabe wird erfindungsgemäß gelöst durch eine Vorrichtung zur Unterstützung von Bewegungen und Hebebewegungen des menschlichen Körpers, insbesondere ein passives Exoskelett, mit den Merkmalen des Patentanspruchs 1.

Vorteilhafte Ausgestaltungen und Weiterbildungen sind in den abhängigen Ansprüchen angegeben.

Die erfindungsgemäße Vorrichtung zur Unterstützung von Bewegungen und Hebebewegungen des menschlichen Körpers, welche am Körper tragbar ist, mit einem Hüftelement, mit zwei Schulterbändern, mit einem Rückenelement, welches an dem Hüftelement angeordnet ist, und mit zwei Beinbändern, welche derart angeordnet sind, dass sie im an den Körper angelegten Zustand der Vorrichtung am Gesäß des Körpers entlang verlaufen, zeichnet sich dadurch aus, dass das Hüftelement einen Versteifungsabschnitt mit einem ersten Endbereich und einem zweiten Endbereich aufweist, welcher im an einen Körper angelegten Zustand der Vorrichtung im unteren Rückenbereich des Körpers zu liegen kommt, dass das Rückenelement einen bügelartigen Abschnitt mit einem ersten Endbereich und einem zweiten Endbereich aufweist, wobei der ersten Endbereich des bügelartigen Abschnitts relativ zu dem ersten Endbereich des Versteifungsabschnitts und der zweite Endbereich des bügelartigen Abschnitts relativ zu dem zweiten Endbereich des Versteifungsabschnitts fixiert ist, derart, dass im an den Körper angelegten Zustand der Vorrichtung der bügelartige Abschnitt auf der vom Körper abgewandten Seite des Versteifungsabschnitts angeordnet ist und der erste Endbereich und der zweite Endbereich des bügelartigen Abschnitts auf unterschiedlichen Seiten der Wirbelsäule des Körpers angeordnet sind.

Als Hüftelement soll im Folgenden ein Element verstanden werden, das im an den Körper einer Person angelegten Zustand der Vorrichtung im Bereich der Hüfte an der Person anliegt und sich insbesondere um den Umfang der Person erstreckt. Es kann, muss aber nicht zwingenderweise, zur Befestigung der Vorrichtung an der Person ausgelegt sein.

Als Rückenelement soll im Folgenden ein Element verstanden werden, dass zumindest abschnittsweise im an den Körper einer Person angelegten Zustand der Vorrichtung am Rücken der Person anliegt.

Als Schulterbänder werden im Folgenden die Elemente verstanden, die das Rückenelement im an den Körper einer Person angelegten Zustand der Vorrichtung am Rücken der Person anliegend fixieren. Sie können vom Rücken über die Schulter auf die Brust der Person und unterhalb der Arme wieder zum Rücken geführt verlaufen. Es ist aber auch möglich dass das Rückenelement sowohl am Rücken als auch auf den Schultern aufliegt und die Schulterbänder nicht auf der Schulter selbst, sondern im Wesentlichen auf der Brust der Person verlaufen.

Der erfindungsgemäße Versteifungsabschnitt weist die Funktion auf, die relativ zueinander fixierten ersten Endbereiche zu den relativ zueinander fixierten zweiten Endbereichen beabstandet zu halten, wobei er sich biegen kann und wirkende Kräfte über die Fläche des Versteifungsabschnitts verteilen kann.

Der erfindungsgemäße bügelartige Abschnitt kann derart mit dem Versteifungsabschnitt zusammenwirken, dass bei auf den bügelartigen Abschnitt wirkender Druckkraft der bügelartige Abschnitt auseinander gepresst wird und dadurch den Versteifungsabschnitt spannt, wobei sich dieser der Form des unteren Rückens anpasst. Die Druckkraft wird dabei flächig, insbesondere über den gesamten Versteifungsabschnitt, auf den Körper der Person eingeleitet, wodurch punktuelle Druckkräfte reduziert werden. Dadurch, dass die Druckkraft im Wesentlichen nur hinten auf den Beckenkamm eingeleitet wird, kann eine zusätzliche Belastung des Iliosakralgelenks nahezu ausgeschlossen und die Gefahr einer Hyperkyphose verringert werden.

Gemäß einer bevorzugten Ausführungsform der Erfindung ist der Versteifungsabschnitt durch einen Textilgewebeabschnitt, welcher insbesondere eine geringe Dehnung aufweist, ausgebildet. Dadurch kann der Versteifungsabschnitt in das Hüftelement integriert werden. Insbesondere ist auch möglich, das Hüftelement vollständig aus dem Textilgewebe zu fertigen.

Gemäß einer besonders bevorzugten Ausführungsform der Erfindung ist der Versteifungsabschnitt als Kunststoff- oder Metallplatte ausgebildet. Ein derartiges Versteifungselement weist Vorteile bei der Reinigung der Vorrichtung auf.

Vorzugsweise weist der Versteifungsabschnitt Aufnahmeöffnungen auf, in welche an dem bügelartigen Abschnitt angeordnete Verbindungsvorsprünge einsetzbar sind. Eine derartige Ausgestaltung ermöglicht auf einfache Art und Weise eine relative Fixierung von Versteifungsabschnitt und bügelartigem Abschnitt.

Gemäß einer bevorzugten Weiterbildung der Erfindung ist der Versteifungsabschnitt auswechselbar an dem Hüftelement angeordnet, vorzugsweise in eine an dem Hüftelement angeordnete Tasche eingeschoben. Dies ermöglicht eine verbesserte Reinigung der Vorrichtung.

Vorteilhafterweise ist der bügelartige Abschnitt an dem Hüftelement mittels Fixierelementen, beispielsweise mittels zwei Klettbändern, fixierbar.

Vorzugsweise ist das Rückenelement aus Kunststoff gefertigt und weist insbesondere ein E-Modul von mehr als 600 N/mm² auf.

Gemäß einer besonders bevorzugten Ausführungsform der Erfindung ist vorgesehen, dass das Rückenelement ein erstes Teilelement und ein zweites Teilelement aufweist, wobei die beiden Teilelemente relativ zueinander in einer Längsrichtung verschiebbar beweglich angeordnet sind. Durch die Verschiebbarkeit in Längsrichtung wird eine Längenänderung des Rückenelements ermöglicht. Wesentlich dabei ist, dass die Beweglichkeit auch bei an den Körper einer Person angelegter Vorrichtung gegeben ist und keine Fixierung der beiden Teilelemente des Rückenelements in einer relativen Position zueinander erfolgt. Durch ein derartig ausgebildetes Rückenelement kann die radiale Längung des Rückens bei einem Beugevorgang ausgeglichen werden. Weiterhin kann dadurch Druck auf den Schultern vermieden werden, wenn beim Überbeugen oder nach vorne gestreckten Armen die Schultern nach vorne geschoben werden.

Gemäß einer bevorzugten Ausführungsform der Erfindung weist das erste Teilelement eine Längsnut auf, in welcher ein an dem zweiten Teilelement fixiertes Führungselement beweglich gelagert ist. Dadurch kann auf einfache Art und Weise eine bewegliche Längenverstellbarkeit, welche insbesondere auch auf geringe Bewegungen der die Vorrichtungen tragenden Person reagieren kann, des Rückenelements erreicht werden.

Vorzugsweise sind die Schulterbänder an dem einen der beiden Teilelemente, insbesondere an dem ersten Teilelement, und der bügelartige Abschnitt an dem anderen der beiden Teilelemente, insbesondere an dem zweiten Teilelement, angeordnet. Dies kann zu einer verbesserten Längenverstellbarkeit beitragen.

Vorteilhafterweise sind die Schulterbänder lösbar an dem Rückenelement angeordnet. Dies ermöglicht eine verbesserte Reinigung der Vorrichtung, da die Schulterbänder separat gewaschen werden können.

Um eine optimale Anpassung der Vorrichtung an den Körper einer Person ermöglichen zu können, sind die Schulterbänder in ihrer Länge vorzugsweise verstellbar ausgebildet.

Eine verbesserte Anpassung der Vorrichtung an den Körper einer Person kann auch erreicht werden, wenn gemäß einer bevorzugten Ausführungsform der Erfindung das Hüftelement als vorzugsweise in der Weite verstellbarer Hüftgurt ausgebildet ist.

Eine bevorzugte Weiterbildung der Erfindung sieht vor, dass die Beinbänder lösbar an dem Hüftelement befestigt sind, insbesondere jedes der Beinbänder lösbar an dem Hüftelement befestigt ist. Dies ermöglicht eine verbesserte Reinigung der Vorrichtung.

Vorzugsweise ist jedes der Beinbänder in der Länge verstellbar ausgebildet, um eine optimale Anpassung der Vorrichtung an den Körper einer Person ermöglichen zu können.

Gemäß einer bevorzugten Ausführungsform der Erfindung ist jedes der Beinbänder an der Außenseite des Hüftelements befestigbar ist, beispielsweise mittels einer Schnalle, einer Gürtelschnalle oder eines Klettverschlusses. Dies ermöglicht ein einfaches Anlegen der Beinbänder.

Zur verbesserten Führung der Beinbänder ist dabei vorzugsweise vorgesehen, dass jedes der Beinbänder mit einem freien Ende durch zwei im Winkel, vorzugsweise im Winkel von 45°, zueinander verlaufende Schlitze, welche insbesondere in dem bügelartigen Abschnitt des Rückenelements angeordnet sind, geführt ist.

Sind dabei vorzugsweise die Beinbänder aus Elastomer gefertigt, ermöglicht dies ein Anlegen der Beinbänder unter einer gewünschten Vorspannung.

Gemäß einer bevorzugten alternativen Ausführungsform der Erfindung sind die beiden Beinbänder direkt miteinander verbunden, gehen vorzugsweise einstückig ineinander über, und sind durch eine Umlenkvorrichtung, welche beispielsweise als Umlenkschnalle ausgebildet sein kann, geführt. Dadurch, dass die beiden Beinbänder verbunden und in der Umlenkvorrichtung gleitend gelagert sind, können Zugbewegungen an den Beinbändern ausgeglichen werden, wodurch das Laufen sowie das Treppensteigen erheblich erleichtert werden kann.

Dabei kann vorzugsweise die Umlenkvorrichtung an einer Rückholvorrichtung angeordnet sein, was eine Unterstützung der Aufrichtbewegung ermöglicht. Die Rückholvorrichtung kann dabei entweder mechanisch ausgebildet, beispielsweise auf einer Rückholfeder basierend, oder auch elektromotorisch angetrieben sein.

Vorzugsweise ist dabei die Umlenkvorrichtung geführt verschiebbar an dem Rückenelement angeordnet, um eine Stabilisierung der Bewegung ermöglichen zu können.

Gemäß einer vorteilhaften Weiterbildung sind in dieser Ausführungsform der Erfindung die Beinbänder durch einen in dem Rückenelement angeordneten, insbesondere quer zur Längsrichtung der Wirbelsäule verlaufenden, Querschlitz geführt. Dadurch kann ein Wegrutschen der Beinbänder seitlich über das Gesäß vermieden werden.

In dieser Ausführungsform der Erfindung können die Beinbänder aus einem Textilmaterial oder aus Elastomer gefertigt sein.

Vorzugsweise weist das Rückenelement ein Nachrüstelement auf, an welchem die Umlenkvorrichtung angeordnet ist. Dadurch wird auf einfache Art und Weise ein Umrüsten oder Nachrüsten von einer Ausführungsform der Erfindung auf die alternative Ausführungsform der Erfindung möglich.

Gemäß einer bevorzugten Ausgestaltung der Erfindung weist jedes der Beinbänder ein Knieelement, insbesondere mit einer oberen Knieschlaufe, einer unteren Knieschlaufe und einem vorzugsweise die Kniescheibe freilassenden Kniescheibenelement, sowie ein Fußelement, insbesondere mit einer Knöchelschlaufe und einer Sohlenschlaufe, auf. Derartig ausgestaltete Beinbänder ermöglichen eine gute Stabilisierung der Beine, insbesondere der Knie, und können einfach angelegt werden.

Die Erfindung wird anhand der nachfolgenden Figuren ausführlich erläutert. Es zeigen
- Figur 1: eine perspektivische Ansicht von schräg vorne eines ersten Ausführungsbeispiels einer erfindungsgemäßen Vorrichtung,
- Figur 2: eine perspektivische Ansicht von schräg hinten der Vorrichtung gemäß Figur 1,
- Figur 3: eine Explosionsdarstellung der Vorrichtung gemäß Figur 1,
- Figur 4: eine Ansicht von hinten auf die Vorrichtung gemäß Figur 1,
- Figur 5: eine Ansicht von vorne auf die Vorrichtung gemäß Figur 1,
- Figur 6: eine perspektivische Ansicht der Vorrichtung gemäß Figur 1 im an den Körper einer Person angelegten Zustand,
- Figur 7: eine Ansicht von vorne auf ein zweites Ausführungsbeispiel einer erfindungsgemäßen Vorrichtung,
- Figur 8: eine Explosionsdarstellung eines Teils der Vorrichtung aus Figur 7 und
- Figur 9: eine schematische Darstellung der wirkenden Kräfte bei der Vorrichtung gemäß Figur 1 und Figur 7.

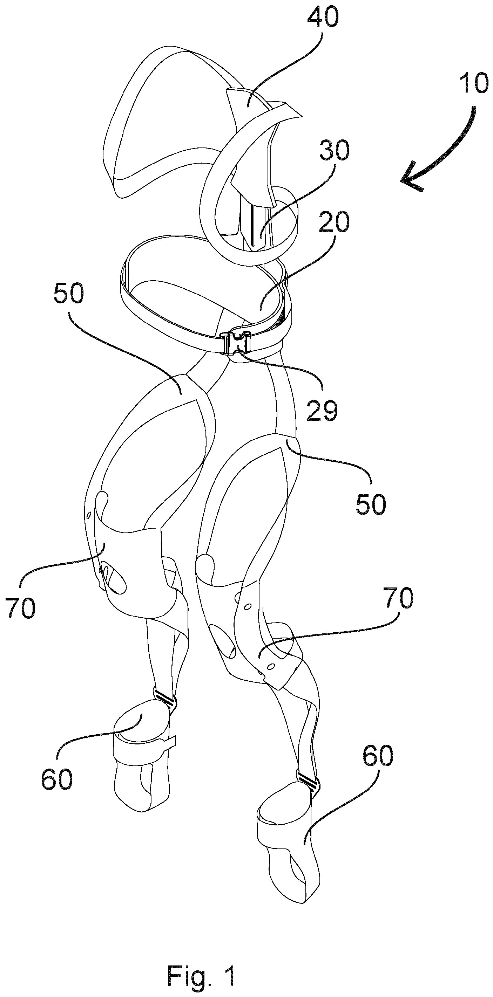

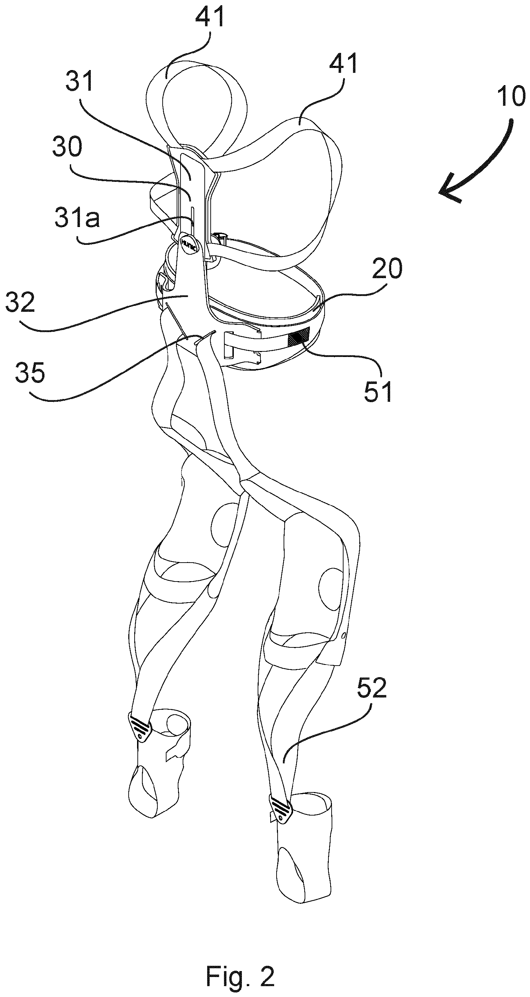

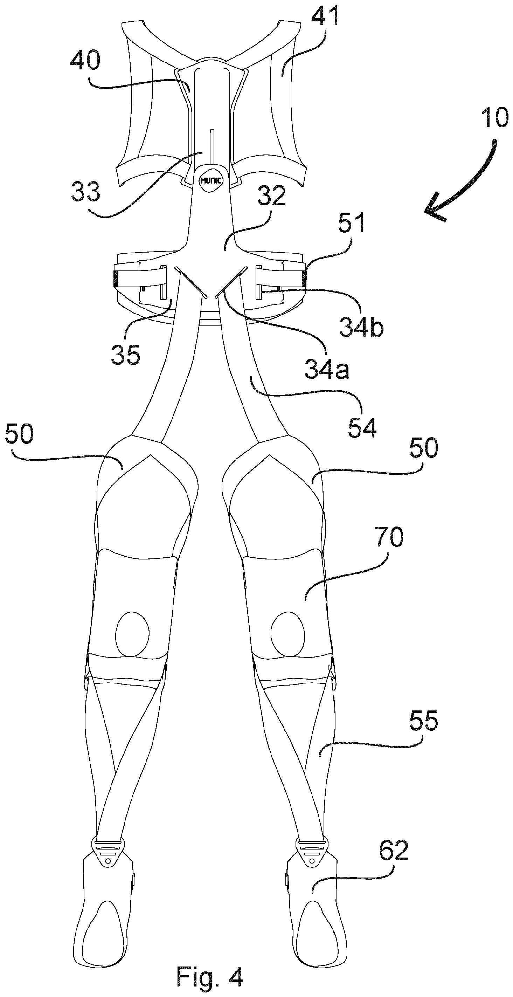

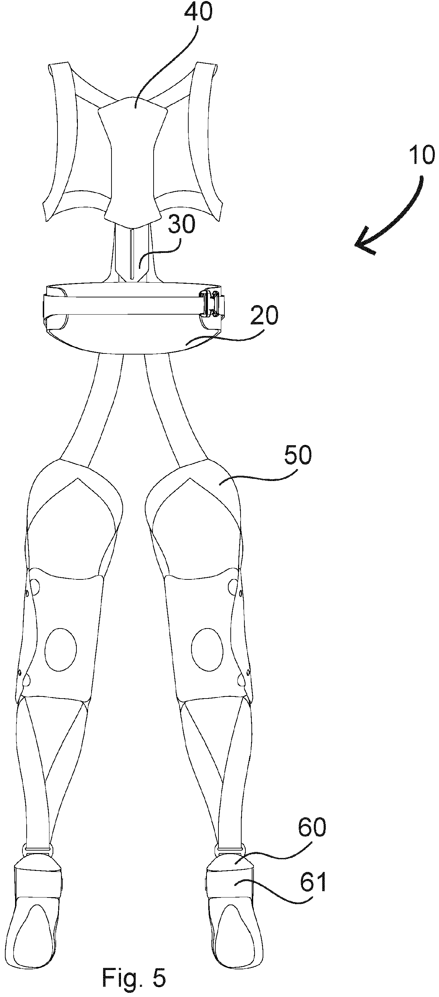

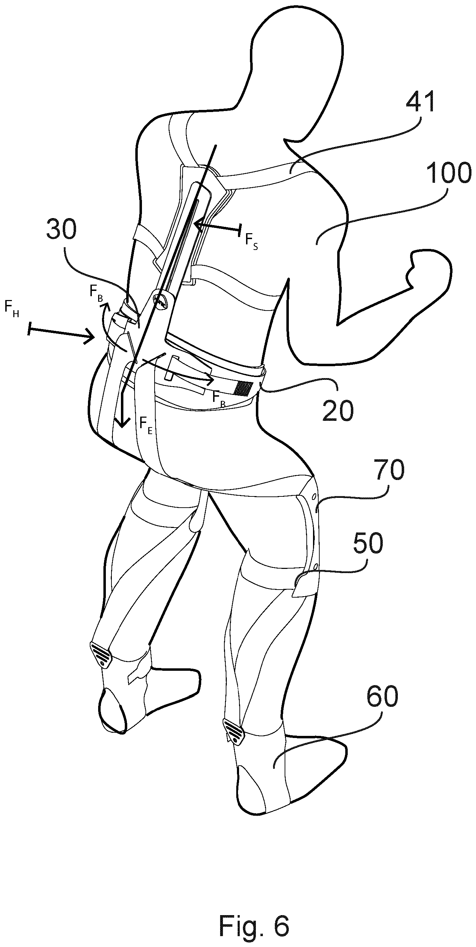

Die Figuren 1 bis 6 zeigen verschiedene Ansichten eines ersten Ausführungsbeispiels einer erfindungsgemäßen Vorrichtung 10 zur Unterstützung von Bewegungen und Hebebewegungen des menschlichen Körpers, welche am Körper tragbar ist (vgl. Figur 6). Zur besseren Übersicht sind nicht sämtliche Bezugsziffern in sämtlichen Figuren angegeben.

Die Vorrichtung 10 weist ein Hüftelement 20 auf, welches beispielsweise als Hüftgurt ausgebildet sein kann. Das Hüftelement 20 kann im Bereich der Hüfte an einer Person 100 fixiert werden (vgl. Figur 6), beispielsweise, indem es die Hüfte umgreift. Zur Vereinfachung des Anlegens kann das Hüftelement 20 einen Verschluss, beispielsweise eine Schnappschnalle 29 oder alternativ auch einen Verschluss nach Art eines Gürtels oder einen Klettverschluss, aufweisen (vgl. Figur 1). Dabei ist insbesondere ein Ende des Hüftelements 20 längenverstellbar an dem entsprechenden Verschluss angeordnet, um die Weite des Hüftelements 20 verstellen zu können.

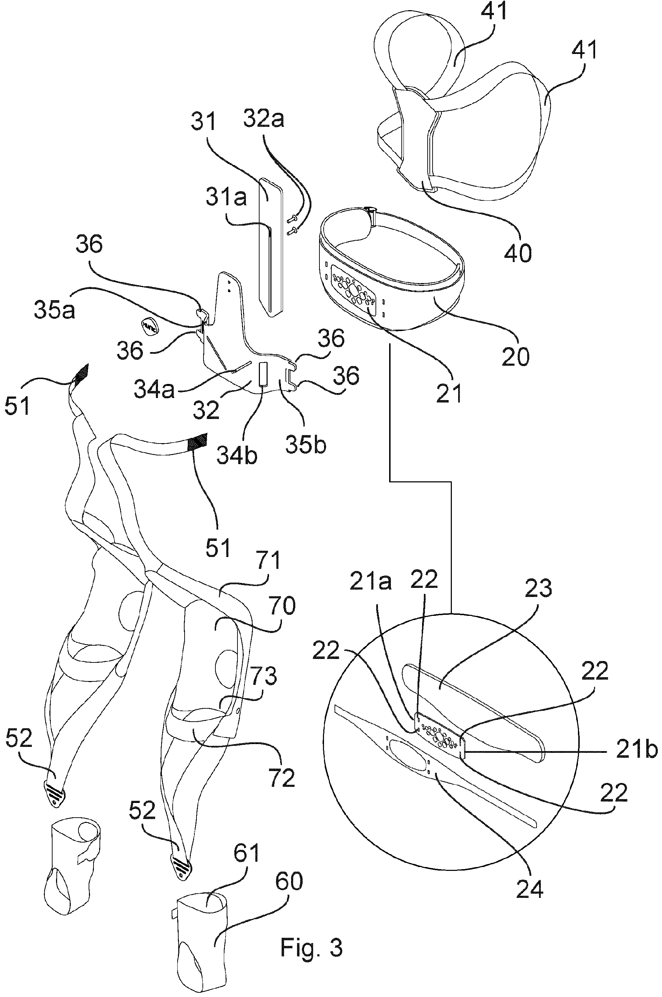

Das Hüftelement 20 weist einen Versteifungsabschnitt 21 mit einem ersten Endbereich 21a und einem zweiten Endbereich 21b auf. Der Versteifungsabschnitt 21 kann in das Hüftelement 20 integriert sein, indem beispielsweise das Hüftelement 20 aus einem entsprechend steifen Material wie beispielsweise einem Textilgewebe abschnittsweise oder vollständig gefertigt ist. Gemäß dem dargestellten Ausführungsbeispiel ist der Versteifungsabschnitt 21 als separates Versteifungselement beispielsweise in Form einer Platte, welche aus Kunststoff oder Metall gefertigt sein kann, ausgebildet, welches an oder in dem Hüftelement 20 angeordnet ist. Der Versteifungsabschnitt 21 kann dazu beispielsweise in eine an dem Hüftelement 20 angeordnete Tasche eingeschoben werden, um auf diese Weise lösbar an dem Hüftelement 20 fixiert werden zu können. Die Tasche kann dadurch ausgebildet sein, dass das Hüftelement 20 zweilagig mit einer ersten Lage 23 und einer zweiten Lage 24 ausgebildet ist (vgl. Ausschnittvergrößerung in Figur 3), welche miteinander verbunden, beispielsweise vernäht, sind derart, dass sich eine Tasche bildet.

Der Versteifungsabschnitt 21 kann Aufnahmeöffnungen 22, beispielsweise in dem ersten Endbereich 21a ein oder, wie in Figur 3 dargestellt, zwei Aufnahmeöffnungen 22, und in dem zweiten Endbereich 21b eine oder, wie in Figur 3 dargestellt, zwei Aufnahmeöffnungen 22 aufweisen.

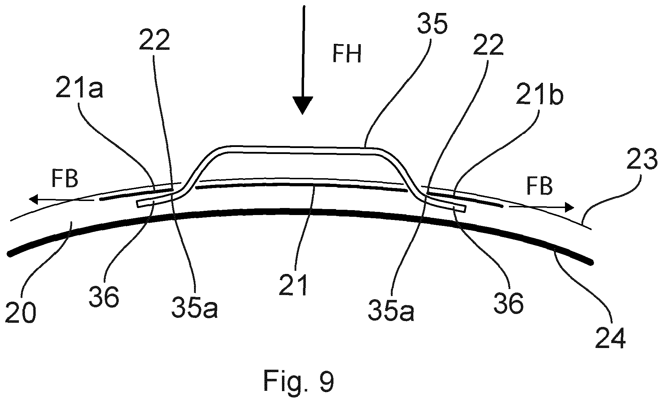

An dem Hüftelement 20 ist ein Rückenelement 30 angeordnet. Das Rückenelement 30 weist einen bügelartigen Abschnitt 35 mit einem ersten Endbereich 35a und einem zweiten Endbereich 35b auf, wobei der erste Endbereich 35a des bügelartigen Abschnitts 35 relativ zu dem ersten Endbereich 21a des Versteifungsabschnitts 21 und der zweite Endbereich 35b des bügelartigen Abschnitts 35 relativ zu dem zweiten Endbereich 21b des Versteifungsabschnitts 21 fixiert ist. Die Fixierung erfolgt derart, dass im an den Körper angelegten Zustand der Vorrichtung 10 der bügelartige Abschnitt 35 auf der vom Körper abgewandten Seite des Versteifungsabschnitts 21 angeordnet ist und der erste Endbereich 35a und der zweite Endbereich des bügelartigen Abschnitts 35 auf unterschiedlichen Seiten der Wirbelsäule des Körpers angeordnet sind (vgl. Figur 6). Im vorliegenden Ausführungsbeispiel erfolgt die Fixierung zwischen dem Versteifungsabschnitt 21 und dem bügelartigen Abschnitt 35 dadurch, dass an dem bügelartigen Abschnitt 35 angeordnete Verbindungsvorsprünge 36 in die an dem Versteifungselement 21 angeordneten Aufnahmeöffnungen 22 eingesetzt werden (vgl. Figur 3 und Figur 9). Dadurch kann eine direkte Fixierung zwischen dem Versteifungsabschnitt 21 und dem bügelartigen Abschnitt 35 erreicht werden. Alternativ ist auch eine indirekte relative Fixierung zwischen dem Versteifungsabschnitt 21 und dem bügelartigen Abschnitt 35 möglich, indem sowohl der Versteifungsabschnitt 21 als auch der bügelartige Abschnitt 35 an dem Hüftelement 20 fixiert werden. Bei einer direkten Fixierung zwischen dem Versteifungsabschnitt 21 und dem bügelartigen Abschnitt 35 kann auch der bügelartige Abschnitt 35 zusätzlich an dem Hüftelement 20 mittels Fixierelementen, beispielsweise mittels zwei Klettbändern, fixiert werden.

Der erfindungsgemäße Versteifungsabschnitt 21 weist die Funktion auf, die relativ zueinander fixierten ersten Endbereiche 21a, 35a zu den relativ zueinander fixierten zweiten Endbereichen 21b, 35b beabstandet zu halten, wobei er sich biegen kann und wirkende Kräfte über die Fläche des Versteifungsabschnitts 21 verteilen kann.

Wie in Figur 9 schematisch dargestellt, kann der erfindungsgemäße bügelartige Abschnitt 35 derart mit dem Versteifungsabschnitt 21 zusammenwirken, dass bei auf den bügelartigen Abschnitt 35 wirkender Druckkraft FH der bügelartige Abschnitt 35 auseinander gepresst wird und dadurch den Versteifungsabschnitt 21 spannt, wobei sich dieser der Form des unteren Rückens anpasst. Die Druckkraft FH wird dabei flächig in Form von Kräften FB, insbesondere über den gesamten Versteifungsabschnitt 21, auf den Körper der Person eingeleitet, wodurch punktuelle Druckkräfte reduziert werden.

Ausgehend von dem bügelartigen Abschnitt 35 erstreckt sich das Rückenelement 30 im an die Person 100 angelegten Zustand etwa in Richtung parallel zur Wirbelsäule vorzugsweise bis in die Nähe der Schulterblätter.

Das Rückenelement 30 kann zumindest abschnittsweise, vorzugsweise vollständig, aus einem formelastischen Material gefertigt sein. Das formelastische Material kann beispielsweise Metall oder Kunststoff sein, wobei Kunststoff aufgrund des geringeren Gewichts in der Regel bevorzugt wird. Vorzugsweise weist das Rückenelement 30 ein E-Modul von mehr als 600 N/mm², vorzugsweise von mehr als 1000 N/mm², beispielsweise ein E-Modul von 5000 N/mm², auf, um eine ausreichende Steifigkeit zur Stabilisierung des Rückens der Person 100 aufweisen zu können.

An dem Rückenelement 30 ist ein Schulterelement 40 mit zwei Schulterbändern 41 angeordnet. Die Schulterbänder 41 können aus einem Elastomer gefertigt sein, das Schulterelement 40 kann aus einem Textilgewebe gefertigt sein. Die Schulterbänder 41 verlaufen im an die Person 100 angelegten Zustand der Vorrichtung 10 vom Rücken über die Schultern zur Brust und von dort unterhalb der Arme wieder zurück auf den Rücken der Person 100. Die Schulterbänder 41 können längenverstellbar ausgebildet sein, um sie an den Körper der Person 100 anpassen zu können. Sind die Schulterbänder 41 aus einem Elastomer gefertigt, genügt gegebenenfalls auch die Elastizität der Schulterbänder 41, um diese an den Körper der Person 100 anzupassen. Das Elastomer kann insbesondere ein E-Modul von weniger als 600 N/mm², vorzugsweise von weniger als 150 N/mm², beispielsweise ein E-Modul von etwa 30 N/mm², aufweisen.

Das Schulterelement 40 ist insbesondere lösbar an dem Rückenelement 30 angeordnet, um dieses separat waschen zu können. Dazu kann das Rückenelement 30 beispielsweise in eine an dem Schulterelement 40 angeordnete Tasche eingeschoben und in dieser fixiert oder an dem Schulterelement 40 fixiert werden.

Das Rückenelement 30 kann ein erstes Teilelement 31 und ein zweites Teilelement 32 aufweisen, welche relativ zueinander in einer Längsrichtung verschiebbar beweglich angeordnet sind. Dazu kann das erste Teilelement 31 beispielsweise eine Längsnut 31a aufweisen, in welcher ein an dem zweiten Teilelement 32 fixiertes Führungselement 32a beweglich gelagert ist. Das Führungselement 32a kann beispielsweise zwei an dem zweiten Teilelement 32 fixierte Stifte umfassen, wobei durch zwei Stifte eine Verdrehsicherung der Verschiebebewegung zwischen den beiden Teilelementen 31, 32 sichergestellt wird.

Das Schulterelement 40 kann bei der zweiteiligen Ausgestaltung des Rückenelements 30 insbesondere an dem ersten Teilelement 31 angeordnet sein, während der bügelartige Abschnitt 43 an dem zweiten Teilelement 32 angeordnet sein kann.

An dem Hüftelement 20 sind zwei Beinbänder 50 derart angeordnet, dass sie im an den Körper angelegten Zustand der Vorrichtung 10 am Gesäß des Körpers der Person 100 entlang verlaufen. Die Beinbänder 50 weisen ein erstes Ende 51 und ein zweites Ende 52 auf. Die Beinbänder 50 sind vorzugweise lösbar mit dem ersten Ende 51 an dem Hüftelement 20, insbesondere jedes der Beinbänder 50 lösbar an dem Hüftelement 20 angeordnet.

Bei dem in den Figuren 1 bis 6 dargestellten ersten Ausführungsbeispiel der Erfindung ist jedes der Beinbänder 50 mit seinem ersten Ende 51 an der Außenseite des Hüftelements 20 befestigbar, beispielsweise mittels einer Schnalle, einer Gürtelschnalle oder besonders bevorzugt mittels eines Klettverschlusses. Derartige Befestigungsmittel sind einfach zu handhaben und erlauben die Befestigung in einer gewünschten Länge, so dass sich eine Längenverstellbarkeit der Beinbänder 50 auf einfache Art und Weise ergibt.

Zur Führung und Positionierung der Beinbänder 50 können insbesondere in dem bügelartigen Abschnitt des Rückenelements Schlitze angeordnet sein. Beispielsweise kann jedes der Beinbänder 50 durch zwei im Winkel, vorzugsweise im Winkel von 45° zueinander verlaufende Schlitze 34a, 34b geführt sein. Damit das Beinband 50, welches unterhalb des Hüftgurts 20 im angelegten Zustand im wesentlichen vertikal verläuft, derart an dem Hüftgurt 20 befestigt werden kann, dass sein erstes Ende 51 im wesentlichen horizontal verläuft, kann beispielsweise der erste Schlitz 34a im Winkel von 45° gegen die Horizontale geneigt und der zweite Schlitz 34b im Wesentlichen vertikal angeordnet sein, um eine entsprechende Umlenkung zu erreichen.

Sind die Beinbänder 50 aus Elastomer gefertigt, können sie unter der gewünschten Vorspannung am Hüftelement 20 befestigt werden. Das Elastomer kann insbesondere ein E-Modul von weniger als 600 N/mm², vorzugsweise von weniger als 150 N/mm², beispielsweise ein E-Modul von etwa 30 N/mm², aufweisen.

An dem zweiten Ende 52 des Beinbands 50 kann ein Fußelement 60 zur Befestigung des zweiten Endes 52 des Beinbands 50 an einem Fuß der Person 100 angeordnet sein. Das Fußelement 60 kann lösbar an dem Beinband 50 befestigt sein, beispielsweise mittels eines Klettverschlusses. Das Fußelement 60 kann eine Knöchelschlaufe 61 und eine Sohlenschlaufe 62 aufweisen. Das Fußelement 60 kann derart über den Fuß der Person 100 gestreift werden, dass die Knöchelschlaufe 61 den Knöchel umschließt und die Sohlenschlaufe 62 sich von der Knöchelschlaufe ausgehend um die Fußsohle legt. Das Anlegen des Fußelements 60 wird vereinfacht, wenn die Knöchelschlaufe öffenbar und wiederverschließbar ist, beispielsweise mittels eines Klettverschlusses oder eines Magnetverschlusses.

Das Beinband 50 kann ein Knieelement 70 aufweisen. Das Knieelement 70 kann eine obere Knieschlaufe 71, eine untere Knieschlaufe 72 und ein Kniescheibenelement 73 aufweisen, wobei das Kniescheibenelement 73 vorzugsweise derart ausgebildet ist, dass es ein Loch aufweist und im an die Person 100 angelegten Zustand der Vorrichtung 10 die Kniescheibe umfassend ausgebildet ist. Im an die Person 100 angelegten Zustand der Vorrichtung 10 umgreift die obere Knieschlaufe 71 das Bein der Person 100 oberhalb des Knies, wobei die obere Knieschlaufe 71 gegen die Längsachse des Oberschenkels der Person 100 geneigt angeordnet sein kann. Die untere Knieschlaufe 72 umgreift im an die Person 100 angelegten Zustand der Vorrichtung 10 das Bein der Person 100 unterhalb des Knies, wobei auch die untere Knieschlaufe 72 gegen die Längsachse des Unterschenkels der Person 100 geneigt angeordnet sein kann.

Die obere Knieschlaufe 71 und die untere Knieschlaufe 72 sind vorzugsweise öffenbar und wiederverschließbar, beispielsweise mittels eines Klettverschlusses oder eines Magnetverschlusses, welcher insbesondere am Kniescheibenelement 73 angeordnet ist, um das Anlegen des Beinbands 50 zu erleichtern.

Das Beinband 50 kann zweigeteilt mit einem ersten Abschnitt 54, welcher das erste Ende 51 umfasst, und einem zweiten Abschnitt 55, welcher das zweite Ende 52 umfasst, ausgebildet sein, wobei das Knieelement 70 zwischen dem ersten Abschnitt 54 und dem zweiten Abschnitt 655 ausgebildet ist. Der zweite Abschnitt 55 kann längenverstellbar ausgebildet sein.

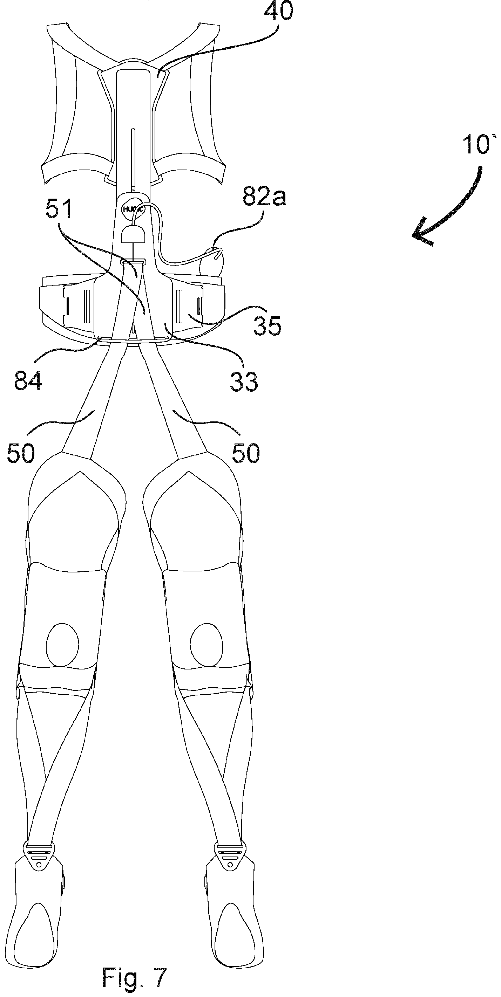

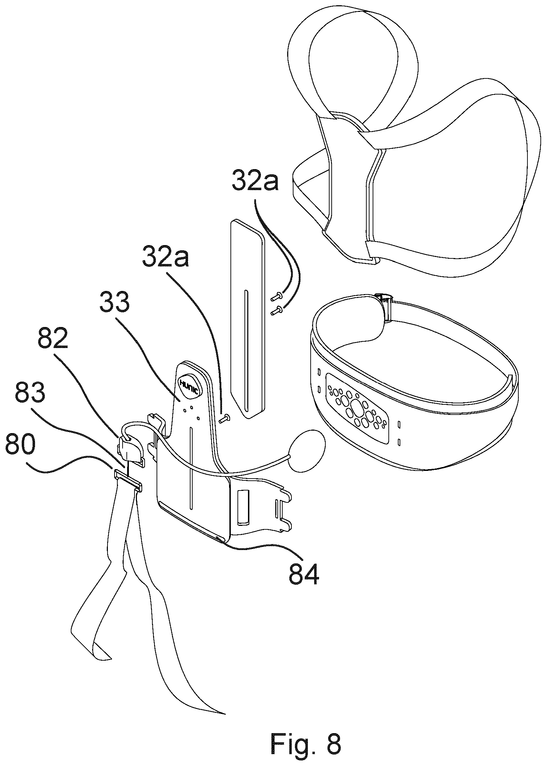

Das in den Figuren 7 und 8 dargestellte zweite Ausführungsbeispiel einer erfindungsgemäßen Vorrichtung 10' unterscheidet sich von der in den Figuren 1 bis 6 dargestellten Vorrichtung 10 in der Befestigung der Beinbänder 50 an dem Hüftelement 20. Gleiche Bezugsziffern bezeichnen gleiche oder funktionsgleiche Teile wie im ersten Ausführungsbeispiel, zur besseren Übersicht sind nicht sämtliche Bezugsziffern in sämtlichen Figuren angegeben.

Bei diesem Ausführungsbeispiel sind die beiden Beinbänder 50 direkt miteinander verbunden, insbesondere an ihren ersten Enden 51, welche vorzugsweise einstückig ineinander übergehen. Dabei sind die Beinbänder 50 durch eine Umlenkvorrichtung 80 geführt, welche beispielsweise als Umlenkschnalle ausgebildet sein kann. Diese Umlenkvorrichtung 80 bewirkt, dass eines der Beinbänder 50 vor der Umlenkvorrichtung 80 und das andere der beiden Beinbänder 50 hinter der Umlenkvorrichtung 80 vom Hüftelement 20 ausgehend sich im an die Person 100 angelegten Zustand der Person nach unten erstrecken kann. Eine Zugbewegung an einem der Beinbänder 50 kann durch ein Nachrutschen des anderen Beinbands 50 durch die Umlenkvorrichtung 80 ausgeglichen werden.

Die Umlenkvorrichtung 80 kann geführt verschiebbar an dem Rückenelement 30 angeordnet sein, beispielsweise entlang eines Gewebebands oder entlang einer Längsnut. Dadurch können Längenänderungen ausgeglichen werden. Durch eine Führung wird die Lage der Umlenkvorrichtung 80 jedoch ausreichend stabilisiert, um ein seitliches Wegrutschen der Beinbänder 50 zu verhindern.

Ein seitliches Wegrutschen der Beinbänder 50 kann alternativ oder zusätzlich auch dadurch verringert werden, dass die Beinbänder 50 durch einen Querschlitz 84, der sich insbesondere im unteren Bereich des Rückenelements 30 über nahezu die gesamte Breite des Rückenelements 30 erstreckt, geführt sind.

Die Umlenkvorrichtung 80 ist insbesondere an einer Rückholvorrichtung 82, insbesondere an einem Zugseil 83 der Rückholvorrichtung 82, angeordnet, welche eine Unterstützung der Aufrichtbewegung ermöglicht. Die Rückholvorrichtung 82 kann beispielsweise mechanisch mit einer Rückzugfeder oder elektromotorisch ausgebildet sein. Durch eine elektromotorische Rückholvorrichtung 82, welche insbesondere ein- und ausschaltbar ausgebildet sein kann, kann eine aktive Komponente in die Vorrichtung 10 integriert werden.

Bei diesem Ausführungsbeispiel können die Beinbänder 50 aus einem Textilmaterial oder einem Elastomer gefertigt sein.

Es ist möglich, die Umlenkvorrichtung 80 an dem zweiten Teilelement 32 des Rückenelements 30 gemäß dem ersten Ausführungsbeispiel anzuordnen. Wie in dem zweiten Ausführungsbeispiel dargestellt, weist das Rückenelement 30 der Vorrichtung 10' ein Nachrüstelement 33 auf, welches an dem zweiten Teilelement 32 befestigbar ist, und an welchem die Umlenkvorrichtung 80 sowie vorzugsweise die Rückholvorrichtung 82 und der Querschlitz 84 angeordnet sind. Ein Bedienelement 82a der Rückholvorrichtung 82 kann an dem Hüftelement 20 angeordnet sein, wo es für die Person 100 einfach zugänglich ist.

### Bezugszeichenliste

- 10: Vorrichtung
- 10': Vorrichtung
- 20: Hüftelement
- 21: Versteifungsabschnitt
- 21a: erster Endbereich
- 21b: zweiter Endbereich
- 22: Aufnahmeöffnung
- 23: Lage
- 24: Lage
- 20: Schnappschnalle
- 30: Rückenelement
- 31: erstes Teilelement
- 31a: Längsnut
- 32: zweites Teilelement
- 32a: Führungselement
- 33: Nachrüstelement
- 34a: Schlitz
- 34b: Schlitz
- 35: bügelartiger Abschnitt
- 35a: erster Endbereich
- 35b: zweiter Endbereich
- 36: Verbindungsvorsprung
- 40: Schulterelement
- 41: Schulterband
- 50: Beinband
- 51: erstes Ende
- 52: zweites Ende
- 54: erster Abschnitt
- 55: zweiter Abschnitt
- 60: Fußelement
- 61: Knöchelelement
- 62: Sohlenelement
- 70: Knieelement
- 71: obere Knieschlaufe
- 72: untere Knieschlaufe
- 73: Kniescheibenelement
- 80: Umlenkvorrichtung
- 82: Rückholvorrichtung
- 82a: Bedienelement
- 83: Zugseil
- 84: Querschlitz
- 100: Person

## Patentansprüche

1. Vorrichtung (10, 10') zur Unterstützung von Bewegungen und Hebebewegungen des menschlichen Körpers, welche am Körper tragbar ist, mit einem Hüftelement (20), mit zwei Schulterbändern (41), mit einem Rückenelement (30), welches an dem Hüftelement (20) angeordnet ist, und mit zwei Beinbändern (50),
wobei das Hüftelement (20) einen Versteifungsabschnitt (21) mit einem ersten Endbereich (21a) und einem zweiten Endbereich (21b) aufweist, welcher im an einen Körper angelegten Zustand der Vorrichtung (10, 10') im unteren Rückenbereich des Körpers zu liegen kommt, dass das Rückenelement (30) einen bügelartigen Abschnitt (35) mit einem ersten Endbereich (35a) und einem zweiten Endbereich (35b) aufweist,
**dadurch gekennzeichnet, dass** die zwei Beinbänder (50) derart angeordnet sind, dass sie im an den Körper angelegten Zustand der Vorrichtung (10, 10') am Gesäß des Körpers entlang verlaufen, wobei der erste Endbereich (35a) des bügelartigen Abschnitts (35) relativ zu dem ersten Endbereich (21a) des Versteifungsabschnitts (21) und der zweite Endbereich (35b) des bügelartigen Abschnitts (35) relativ zu dem zweiten Endbereich (21b) des Versteifungsabschnitts (21) fixiert ist, derart, dass im an den Körper angelegten Zustand der Vorrichtung (10, 10') der bügelartige Abschnitt (35) auf der vom Körper abgewandten Seite des Versteifungsabschnitts (21) angeordnet ist und der erste Endbereich (35a) und der zweite Endbereich (35b) des bügelartigen Abschnitts (35) auf unterschiedlichen Seiten der Wirbelsäule des Körpers angeordnet sind.

2. Vorrichtung nach Anspruch 1,
**dadurch gekennzeichnet, dass** der Versteifungsabschnitt (21) durch einen Textilgewebeabschnitt, welcher insbesondere eine geringe Dehnung aufweist, ausgebildet ist.

3. Vorrichtung nach Anspruch 1,
**dadurch gekennzeichnet, dass** der Versteifungsabschnitt (21) als Kunststoff- oder Metallplatte ausgebildet ist.

4. Vorrichtung nach einem der vorhergehenden Ansprüche, **dadurch gekennzeichnet, dass** der Versteifungsabschnitt (21) Aufnahmeöffnungen (22) aufweist, in welche an dem bügelartigen Abschnitt (35) angeordnete Verbindungsvorsprünge (36) einsetzbar sind.

5. Vorrichtung nach einem der vorhergehenden Ansprüche, **dadurch gekennzeichnet, dass** der Versteifungsabschnitt (21) auswechselbar an dem Hüftelement (20) angeordnet ist, vorzugsweise in eine an dem Hüftelement (20) angeordnete Tasche eingeschoben ist.

6. Vorrichtung nach einem der vorhergehenden Ansprüche, **dadurch gekennzeichnet, dass** der bügelartige Abschnitt (35) an dem Hüftelement (20) mittels Fixierelementen, beispielsweise mittels zwei Klettbändern, fixierbar ist.

7. Vorrichtung nach einem der vorhergehenden Ansprüche, **dadurch gekennzeichnet, dass** das Rückenelement (30) aus Kunststoff gefertigt ist und insbesondere ein E-Modul von mehr als 600 N/mm² aufweist.

8. Vorrichtung nach einem der vorhergehenden Ansprüche, **dadurch gekennzeichnet, dass** das Rückenelement (30) ein erstes Teilelement (31) und ein zweites Teilelement (32) aufweist, wobei die beiden Teilelemente (31, 32) relativ zueinander in einer Längsrichtung verschiebbar beweglich angeordnet sind.

9. Vorrichtung nach einem der vorhergehenden Ansprüche, **dadurch gekennzeichnet, dass** das erste Teilelement (31) eine Längsnut (31a) aufweist, in welcher ein an dem zweiten Teilelement (32) fixiertes Führungselement (32b) beweglich gelagert ist.

10. Vorrichtung nach einem der vorhergehenden Ansprüche, **dadurch gekennzeichnet, dass** die Schulterbänder (41) an dem einen der beiden Teilelemente, insbesondere an dem ersten Teilelement (31), und der bügelartige Abschnitt (35) an dem anderen der beiden Teilelemente, insbesondere an dem zweiten Teilelement (32), angeordnet sind.

11. Vorrichtung nach einem der vorhergehenden Ansprüche, **dadurch gekennzeichnet, dass** die Schulterbänder (41) lösbar an dem Rückenelement (30) angeordnet sind.

12. Vorrichtung nach einem der vorhergehenden Ansprüche, **dadurch gekennzeichnet, dass** die Schulterbänder (41) in ihrer Länge verstellbar ausgebildet sind.

13. Vorrichtung nach einem der vorhergehenden Ansprüche, **dadurch gekennzeichnet, dass** das Hüftelement (20) als vorzugsweise in der Weite verstellbarer Hüftgurt ausgebildet ist.

14. Vorrichtung nach einem der vorhergehenden Ansprüche, **dadurch gekennzeichnet, dass** die Beinbänder (50) lösbar an dem Hüftelement (20) befestigt sind, insbesondere jedes der Beinbänder (50) lösbar an dem Hüftelement (20) befestigt ist.

15. Vorrichtung nach einem der vorhergehenden Ansprüche, **dadurch gekennzeichnet, dass** jedes der Beinbänder (50) in der Länge verstellbar ausgebildet ist.

16. Vorrichtung nach einem der vorhergehenden Ansprüche, **dadurch gekennzeichnet, dass** jedes der Beinbänder (50) an der Außenseite des Hüftelements (20) befestigbar ist, beispielsweise mittels einer Schnalle, einer Gürtelschnalle oder eines Klettverschlusses.

17. Vorrichtung nach Anspruch 16,
**dadurch gekennzeichnet, dass** jedes der Beinbänder (50) mit einem freien Ende (51) durch zwei im Winkel, vorzugsweise im Winkel von 45°, zueinander verlaufende Schlitze (34a, 34b), welche insbesondere in dem bügelartigen Abschnitt (35) des Rückenelements (30) angeordnet sind, geführt ist.

18. Vorrichtung nach einem der Ansprüche 16 bis 17, **dadurch gekennzeichnet, dass** die Beinbänder (50) aus Elastomer gefertigt sind.

19. Vorrichtung nach einem der Ansprüche 1 bis 15,
**dadurch gekennzeichnet, dass** die beiden Beinbänder (50) direkt miteinander verbunden sind, vorzugsweise einstückig ineinander übergehen, und durch eine Umlenkvorrichtung (80) geführt sind.

20. Vorrichtung nach Anspruch 19,
**dadurch gekennzeichnet, dass** die Umlenkvorrichtung (80) an einer Rückholvorrichtung (82) angeordnet ist.

21. Vorrichtung nach einem der Ansprüche 19 bis 20,
**dadurch gekennzeichnet, dass** die Umlenkvorrichtung (80) geführt verschiebbar an dem Rückenelement (30) angeordnet ist.

22. Vorrichtung nach einem der Ansprüche 19 bis 21,
**dadurch gekennzeichnet, dass** die Beinbänder (50) durch einen in dem Rückenelement (30) angeordneten, insbesondere quer zur Längsrichtung der Wirbelsäule verlaufenden, Querschlitz (84) geführt sind.

23. Vorrichtung nach einem der Ansprüche 19 bis 22,
**dadurch gekennzeichnet, dass** die Beinbänder (50) aus einem Textilmaterial oder aus Elastomer gefertigt sind.

24. Vorrichtung nach einem der Ansprüche 19 bis 23,
**dadurch gekennzeichnet, dass** das Rückenelement (30) ein Nachrüstelement (33) aufweist, an welchem die Umlenkvorrichtung (80) angeordnet ist.

25. Vorrichtung nach einem der vorhergehenden Ansprüche, **dadurch gekennzeichnet, dass** jedes der Beinbänder (50) ein Knieelement (70), insbesondere mit einer oberen Knieschlaufe (71), einer unteren Knieschlaufe (72) und einem vorzugsweise die Kniescheibe freilassenden Kniescheibenelement (73), sowie ein Fußelement (60), insbesondere mit einer Knöchelschlaufe (61) und einer Sohlenschlaufe (62), aufweist.

## Claims

1. Apparatus (10, 10') for supporting movements and lifting movements of the human body, which can be worn on the body, having a waist element (20) with two shoulder straps (41), and having a back element (30), which is arranged on the waist element (20), and having two leg straps (50),
wherein the waist element (20) comprises a reinforcement section (21), with a first end region (21a) and with a second end region (21b), which, in the state of the apparatus (10, 10') in which it is worn on the body, rests against the lower back region of the body, and wherein the back element (30) comprises a bracket-like section (35), with a first end region (35a) and with a second end region (35b),
**characterized in that** the two leg straps (50) are arranged such that, in the state of the apparatus (10, 10') in which it is worn on the body, they extend along the buttocks of the body, wherein the first end region (35a) of the bracket-like section (35) is fixated relative to the first end region (21a) of the reinforcement section (21), and the second end region (35b) of the bracket-like section (35) is fixated relative to the second end region (21b) of the reinforcement section (21), such that, in the state of the apparatus (10, 10') in which it is worn on the body, the bracket-like section (35) is arranged on the side of the reinforcement section (21) which faces away from the body and the first end region (35a) and the second end region (35b) of the bracket-like section (35) are arranged on opposite sides of the spinal column of the body.

2. Apparatus in accordance with claim 1,
**characterized in that** the reinforcement section (21) is implemented by means of a textile fabric section which, in particular, comprises a minimal elongation.

3. Apparatus in accordance with claim 1,
**characterized in that** the reinforcement section (21) is implemented as a plastic or metal plate.

4. Apparatus in accordance with any of the preceding claims,
**characterized in that** the reinforcement section (21) comprises receptacle apertures, into which connecting projections (36) arranged on the bracket-like section (35) can be inserted.

5. Apparatus in accordance with any of the preceding claims,
**characterized in that** the reinforcement section (21) is interchangeably arranged on the waist element (20), preferably inserted in a pocket arranged on the waist element (20).

6. Apparatus in accordance with any of the preceding claims,
**characterized in that** the bracket-like section (35) can be fixated on the waist element (20) by means of fixation elements, for example, by means of two Velcro straps.

7. Apparatus in accordance with any of the preceding claims,
**characterized in that** the back element (30) is manufactured from plastic and, in particular, comprises an elasticity modulus of more than 600 N/mm².

8. Apparatus in accordance with any of the preceding claims,
**characterized in that** the back element (30) comprises a first partial element (31) and a second partial element (32), wherein the two partial elements (31, 32) are arranged moveably in relation to each other in a longitudinal direction.

9. Apparatus in accordance with any of the preceding claims,
**characterized in that** the first partial element (31) comprises an elongate slot (31a), in which a guide element (32b) fixated on the second partial element (32) is moveably supported.

10. Apparatus in accordance with any of the preceding claims,
**characterized in that** the shoulder straps (41) are arranged on one of the two partial elements, in particular, on the first partial element (31) and **in that** the bracket-like section (35) is arranged on the other one of the two partial elements, in particular, on the second partial element (32).

11. Apparatus in accordance with any of the preceding claims,
**characterized in that** the shoulder straps (41) are detachably arranged on the back element (30).

12. Apparatus in accordance with any of the preceding claims,
**characterized in that** the shoulder straps (41) are implemented to be adjustable in length.

13. Apparatus in accordance with any of the preceding claims,
**characterized in that** the waist element (20) is preferably implemented as a waist belt which is adjustable in width.

14. Apparatus in accordance with any of the preceding claims,
**characterized in that** the leg straps (50) are detachably attached to the waist element (20), in particular, each of the leg straps (50) is detachably attached to the waist element (20).

15. Apparatus in accordance with any of the preceding claims,
**characterized in that** each of the leg straps (50) is implemented to be adjustable in length.

16. Apparatus in accordance with any of the preceding claims,
**characterized in that** each of the leg straps (50) can be attached to the outer side of the waist element (20), for example, by means of a clasp, a belt buckle or a Velcro fastener.

17. Apparatus in accordance with claim 16,
**characterized in that** a free end (51) of each of the leg straps (50) is fed through two slits (34a, 34b), preferably extending at an angle of 45° to each other, which are arranged, in particular, in the bracket-like section (35) of the back element (30).

18. Apparatus in accordance with either of claims 16 or 17,
**characterized in that** the leg straps (50) are made of elastomer.

19. Apparatus in accordance with any of claims 1 to 15,
**characterized in that** the two leg straps (50) are directly connected to each other, preferably integrally transitioning into each other, and are fed through a deflection device (80).

20. Apparatus in accordance with claim 19,
**characterized in that** the deflection device (80) is arranged on a retraction mechanism (82).

21. Apparatus in accordance with either of claims 19 or 20,
**characterized in that** the deflection device (80) is arranged on the back element (30) moveably and in a guided manner.

22. Apparatus in accordance with any of claims 19 to 21,
**characterized in that** the leg straps (50) are fed through a transverse slit (84) arranged, in particular, transversely to the longitudinal direction of the spinal column, on the back element (30).

23. Apparatus in accordance with any of claims 19 to 22,
**characterized in that** the leg straps (50) are manufactured from a textile material or an elastomer.

24. Apparatus in accordance with any of claims 19 to 23,
**characterized in that** the back element (30) comprises a retrofitting element (33) on which the deflection device (80) is arranged.

25. Apparatus in accordance with any of the preceding claims
**characterized in that** each of the leg straps (50) comprises a knee element (70), in particular, with an upper knee loop (71) and a lower knee loop (72), and with a knee cap element (73) which preferably leaves the knee cap exposed, as well as a foot element (60), in particular, having an ankle loop (61) and a sole loop (62).

## Revendications

1. Dispositif (10, 10') pour accompagner des mouvements et des mouvements de levage du corps humain qui est porté par le corps, avec un élément de hanche (20), des bandes d'épaule (41), un élément de dos (30) qui est installé sur l'élément de hanche (20) et deux bandes de jambe (50),
dans lequel
l'élément de hanche (20) a un segment de rigidification (21) avec une première zone d'extrémité (21a) et une seconde zone d'extrémité (21b) qui, lorsque le dispositif (10, 10') est appliqué contre un corps, vient en appui dans la zone basse du dos du corps de façon que l'élément de dos (30) présente un segment en forme d'arceau (35) avec une première zone d'extrémité (35a) et une seconde zone d'extrémité (35b),
dispositif **caractérisé en ce que**
les deux bandes de jambe (50) sont disposées pour qu'elles passent sur l'assise du corps lorsque le dispositif (10, 10') est dans son état appliqué au corps,
dans lequel
la première zone d'extrémité (35a) du segment en forme d'arceau (35) est fixée par rapport à la première zone d'extrémité (21a) du segment de rigidification (21) et la seconde zone d'extrémité (35b) du segment en arceau (35) est fixée par rapport à la seconde zone d'extrémité (21b) du segment de rigidification (21) de façon que dans l'état du dispositif (10, 10') appliqué sur le corps, le segment en forme d'arceau (35) soit sur le côté du segment de rigidification (21) à l'opposé du corps et que la première zone d'extrémité (35a) et la seconde zone d'extrémité (35b) du segment en forme d'arceau (35) se trouvent sur des côtés différents de la colonne vertébrale du corps.

2. Dispositif selon la revendication 1,
**caractérisé en ce que**
le segment de rigidification (21) est un segment de tissu textile ayant notamment un faible allongement.

3. Dispositif selon la revendication 1,
**caractérisé en ce que**
le segment de rigidification (21) est une plaque de matière plastique ou de métal.

4. Dispositif selon l'une des revendications précédentes,
**caractérisé en ce que**
le segment de rigidification (21) a des orifices récepteurs (22) recevant des reliefs de liaison (36) du segment (35) en forme d'arceau.

5. Dispositif selon l'une des revendications précédentes,
**caractérisé en ce que**
le segment de rigidification (21) est installé de manière détachable sur l'élément de hanche (20), de préférence il est glissé dans une poche installée sur l'élément de hanche (20).

6. Dispositif selon l'une des revendications précédentes,
**caractérisé en ce que**
le segment en forme d'arceau (35) se fixe à l'élément de hanche (20) avec des éléments de fixation, par exemple, avec deux bandes à griffes.

7. Dispositif selon l'une des revendications précédentes,
**caractérisé en ce que**
l'élément de dos (30) est fabriqué en matière plastique et il a notamment un module d'élasticité E supérieur à 600 N/m².

8. Dispositif selon l'une des revendications précédentes,
**caractérisé en ce que**
l'élément de dos (30) a une première partie d'élément (31) et une seconde partie d'élément (32), les deux parties d'élément (31, 32) étant installées l'une par rapport à l'autre de façon à coulisser dans la direction longitudinale.

9. Dispositif selon l'une des revendications précédentes,
**caractérisé en ce que**
la première partie d'élément (31) a une rainure longitudinale (31a) recevant, de manière mobile, un élément de guidage (32b) fixé à la seconde partie d'élément (32).

10. Dispositif selon l'une des revendications précédentes,
**caractérisé en ce que**
les bandes d'épaule (41) sont installées notamment sur l'une des deux parties d'éléments notamment sur la première partie d'élément (31) et le segment en forme d'arceau (35) est à l'autre des deux parties d'élément, notamment la seconde partie d'élément (32).

11. Dispositif selon l'une des revendications précédentes,
**caractérisé en ce que**
les bandes d'épaule (41) sont installées de manière amovible sur le premier élément de dos (30).

12. Dispositif selon l'une des revendications précédentes,
**caractérisé en ce que**
les bandes d'épaule (41) sont de longueur réglable.

13. Dispositif selon l'une des revendications précédentes,
**caractérisé en ce que**
l'élément de hanche (20) est de préférence une ceinture de hanche de longueur réglable.

14. Dispositif selon l'une des revendications précédentes,
**caractérisé en ce que**
les bandes de jambe (50) sont fixées de manière amovible à l'élément de hanche (20) et en particulier chacune des bandes de jambe (50) est fixée de manière amovible à l'élément de hanche (20).

15. Dispositif selon l'une des revendications précédentes,
**caractérisé en ce que**
chacune des bandes de jambe (50) est de longueur réglable.

16. Dispositif selon l'une des revendications précédentes,
**caractérisé en ce que**
chacune des bandes de jambe (50) se fixe sur le côté extérieur de l'élément de hanche (20), par exemple, avec une boucle, une boucle de ceinture ou une liaison à griffes.

17. Dispositif selon la revendication 16,
**caractérisé en ce que**
chacune des bandes de jambe (50) est guidée par son extrémité libre (51) à travers deux fentes (34a, 34b) faisant entre elles un angle, de préférence un angle de 45°, ces bandes étant guidées notamment dans un segment (35) en forme d'étrier de l'élément de dos (30).

18. Dispositif selon l'une des revendications 16 à 17,
**caractérisé en ce que**
les bandes latérales (50) sont fabriquées en élastomère.

19. Dispositif selon l'une des revendications 1 à 15,
**caractérisé en ce que**
les deux bandes de jambe (50) sont reliées directement l'une à l'autre, de préférence elles se rejoignent en une seule pièce et sont guidées dans un dispositif de renvoi (80).

20. Dispositif selon la revendication 19,
**caractérisé en ce que**
le dispositif de renvoi (80) est sur un dispositif de rappel (82).

21. Dispositif selon l'une des revendications 19 à 20,
**caractérisé en ce que**
le dispositif de renvoi (80) est monté de manière guidée en coulissement sur l'élément de dos (30).

22. Dispositif selon l'une des revendications 19 à 21,
**caractérisé en ce que**
les bandes de jambe (50) sont guidées dans une fente transversale (84) dans l'élément de dos (30) notamment orientées transversalement à la direction longitudinale de la colonne vertébrale.

23. Dispositif selon l'une des revendications 19 à 22,
**caractérisé en ce que**
les bandes de jambe (50) sont en une matière textile ou en un élastomère.

24. Dispositif selon l'une des revendications 19 à 23,
**caractérisé en ce que**
l'élément de dos (30) a un élément d'équipement en seconde monte (33) recevant le dispositif de renvoi (80).

25. Dispositif selon l'une des revendications précédentes,
**caractérisé en ce que**
chacune des bandes de jambe (50) a un élément de genou (70) notamment avec une boucle de genou supérieure (71), une boucle de genou inférieure (72) et un élément de ménisque de genou (73) qui laisse de préférence dégagés les ménisques ainsi qu'un élément de pied (60) notamment avec une boucle de cheville (61) et une boucle de semelle (62).
